# EUROPEAN PATENT APPLICATION

(11) **EP 3 623 808 A1**
(43) Date of publication of application: **18.03.2020**
(21) Application number: 18194729.2
(22) Date of filing: 17.09.2018
(51) Int. Cl.: G01N 33/00, G01N 31/00, A61B 5/00

(54) **ELECTROCHEMICAL SENSOR FOR THE MEASUREMENT OF WATER CONTENT**

(71) Applicant: Seitz, Peter, 8902 Urdorf (CH)
(72) Inventor: Heeb, Peter, 9436 Balgach (CH); Baur, Monika, 9326 Horn (CH); Cagin, Emine, 9470 Buchs (CH); Bernard, André, 9014 St. Gallen (CH)
(74) Representative: Richter, Allen

(57) **Abstract**

The invention concerns an electrochemical sensor (1) operative to measure water in liquid, gaseous or vapor fluids. The water reacts with a reactant (6) to generate hydrogen gas as a reaction product. By using an electro-chemical transducing element (2A), an electric signal is generated in accordance with a stoichiometric volume of the hydrogen gas.

## Description

### BACKGROUND

High-precision measurement of water content in the flow regime of less than 10 microliter/minute is crucial for many applications. Today, there are multiple state-of-the art solutions; however, each suffers from a deficiency. Many solutions require expensive, specialized equipment that are dependent on an external electrical power source and require a lab setting. Examples include, thermal mass flow meter, Doppler ultrasonic flow meters, Venturi flow meters, Coriolis flow meters just to name a few.

Thermal mass-flow meters are simpler to employ; however, they are limited to a defined continuum of flow of fluid having a constant and well-known density and heat capacitance within the volume stream passing the sensor and are therefore incapable of measuring water flow in vapor (mixed) phase.

Therefore, there is a need for a high-precision water measurement device operative for liquid, gaseous, and vapor states, that is inexpensive, and deployable in a wide variety of settings function in the above-noted flow regime.

### BRIEF DESCRIPTION OF THE DRAWINGS

The figures illustrate generally, by way of example, but not by way of limitation, various embodiments discussed in the present document.

For simplicity and clarity of illustration, elements shown in the figures have not necessarily been drawn to scale. For example, the dimensions of some of the elements may be exaggerated relative to other elements for clarity of presentation. Furthermore, reference numerals may be repeated among the figures to indicate corresponding or analogous elements. References to previously presented elements are implied without necessarily further citing the drawing or description in which they appear. The number of elements shown in the Figures should by no means be construed as limiting and is for illustrative purposes only. The figures are listed below.
**FIG. 1** is a schematic, cross-sectional side-view of an electrochemical sensor, according to an embodiment;
**FIGS. 2A-2F** are schematic, cross-sectional side-views of various embodiments of a transducing element employable in the electrochemical sensor of FIG. 1;
**FIG. 3** is a schematic, cross-sectional side-view of the electrochemical sensor of FIG. 1 mounted on a body to measure water content of perspiration; and
**FIG.** 4 is a flowchart of the process steps employed by the electrochemical sensor, according to an embodiment.

### DETAILED DESCRIPTION

The following description sets forth various details to provide a thorough understanding of the invention and it should be appreciated, by those skilled in the art, that the present invention may be practiced without these specific details. Furthermore, well-known methods, procedures, and components have been omitted to highlight the present invention.

The present invention pertains to a high-precision, water content measuring device implemented as an electrochemical sensor that is operative to measure extremely-low water content in flow or non-flow settings either as a gas, a liquid, or a vapor (mixed state). Therefore, in some embodiments, the electrochemical sensor is operable to perform phase-independent mass-flow measurement of water.

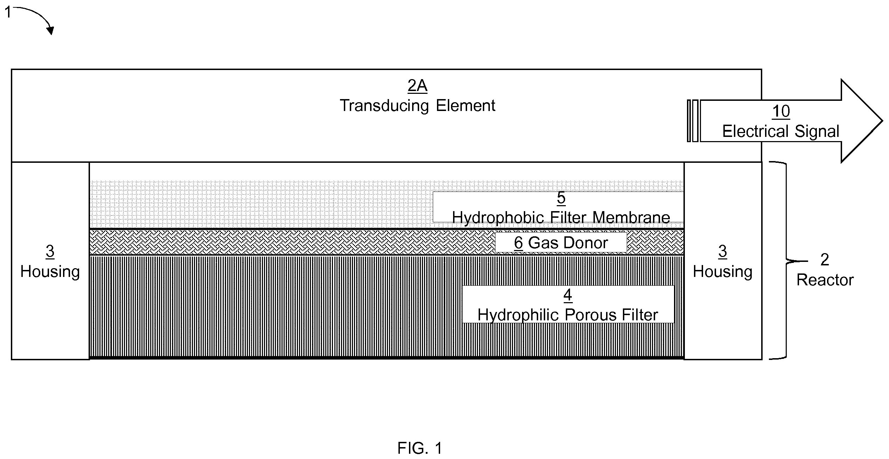

Turning now to the figures, **FIG. 1** is a schematic, cross-sectional side-view of an electrochemical sensor **1** and includes, for example, two primary elements; the reactor **2** in communication with transducing element **2A,** according to an embodiment. Reactor **2** includes, for example, a (e.g., polymeric) housing **3** encasing a hydrophilic porous filter **4** for capturing moisture and filtering impurities, a gas donor **6** characterized by its ability to generate (e.g., hydrogen) gas upon reaction with water, and a hydrophobic filter membrane **5** operative to capture unreacted water and other reactants. Transducing element **2A** is operative to transduce the gas into an electrical signal **10** and can be implemented in various configurations as will be further discussed.

Hydrophilic porous filter **4** provides a constant flow resistance to both liquid and gas states in the above-noted flow regime and the hydrophilic properties advantageously do not hinder passage of liquid water at the low pressures typically associated with low flow rate regimes. Filter **4** may be made, for example, of glass, ceramic, metal, and/or cellulose and may, for example, have a pore size of 450 nm enabling passage of liquid and vapor while filtering particles or salt compounds.

In another embodiment, Hydrophilic porous filter **4** is implemented as Anodic Aluminum Oxide or Anodic Titanium Oxide with a pore size of, e.g., 5-500nm.

As shown, gas donor **6** is disposed at the downstream side of filter **4** to enable reaction with filtered water vapor conveyed by pressure exerted by the water source through primary porous filter **4.** In certain filters **4,** the liquid water is also conveyed through via capillary action from the outside to the inside of upstream reactor **2.**

In some embodiments, CAH₂ is employed as the gas donor. Alternative donors of hydrogen include, for example, metal-hydrides such as MgH₂, NaAlH₄, LiAlH₄, LiH, LiBH₂, LiBH4, non-metal hydrides, and/or some carbohydrates. The gas donor can act as a battery substitute and may define the upper limit of the cumulative electrical power that can be generated.

Hydrophobic filter membrane **5** is implemented as a barrier to enclose the CaH₂, H₂O and Ca(OH)₂ and prevent them from entering the downstream fuel cell, in a certain embodiment. In another embodiment, filter membrane **5** is implemented as combination cellulose/polyester cloth.

Transducing element **2A** translates the gas into an electrical signal by any of a variety of transducing element embodiments, as will be now discussed.

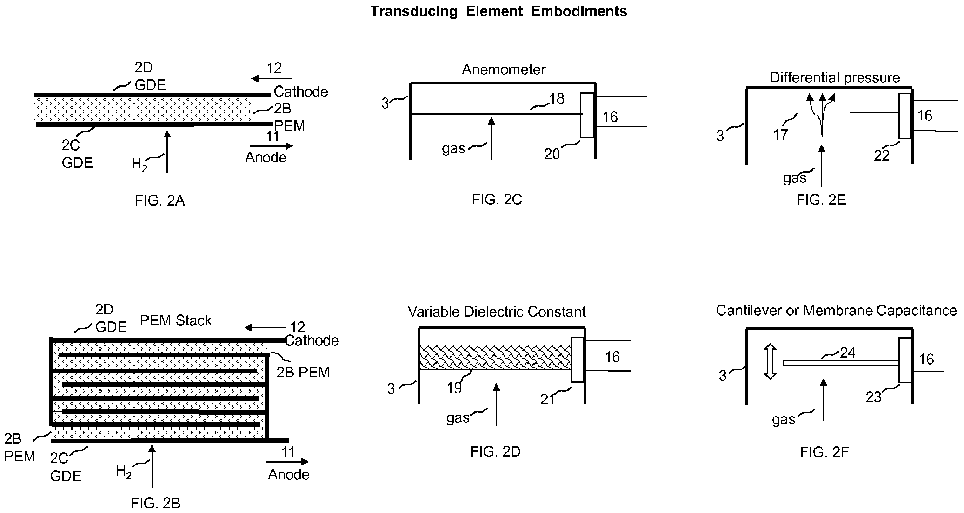

**FIGS. 2A-2F** are schematic, cross-sectional side-views of various embodiments of transducing element **2A.**

Specifically, **FIG. 2A** depicts an embodiment of transducing element **2A** implemented as a proton-exchange membrane (PEM) fuel cell in which hydrogen contacting a downstream gas diffusion electrode (GDE) **2C** is oxidized and the electrons exit the cell on the anode side **11** through an electrical conductor. The resulting cations traverse PEM **2B.** At GDE **2D** the hydrogen ions recombine with electrons and form water through reaction with oxygen. In a certain embodiment PEM **2B** is implemented as Nafion.

**FIG. 2B** depicts an embodiment of transducing element **2A** employing a polymer stack of the PEM fuel cells.

**FIG. 2C** depicts an embodiment of transducing element **2A** that employs a heating filament **18** cooled by the flow of gas. As shown, at least some of the gas is directed over a resistance heated wire **18.** The resulting change in resistance or temperature distribution profile is measured by circuitry **20** and output, e.g., via leads **16** and/or a wireless transmitter. It should be appreciated that the above noted deficiencies of an anemometer are removed through conversion of water vapor, characterized by its composition-dependent density and heat capacity, to a pure gas

**FIG. 2D** depicts an embodiment of transducing element **2A** that employs a porous material **19** whose dielectric constant changes as it fills with gas. The resulting change in capacitance is processed by circuitry **21** and outputs a signal via leads **16** and/or a wireless transmitter. Zeolite is an example of a such a porous material.

**FIG. 2E** depicts an embodiment of transducing element **2A** that employs a differential pressure sensor to transduce gas pressure into an electrical signal. As shown, a differential pressure is created as gas passes through orifice **17** and is transduced into an electric signal by circuitry **22,** and signal output, e.g., via leads **16** and/or a wireless transmitter.

**FIG. 2F** depicts an embodiment of transducing element **2A** that employs a cantilever or stretchable membrane configured to deflect responsively to the local pressure field generated by the gas-stream. As shown, gas applies a pressure to flexible element **24** and the resulting deformation is quantified through an electromechanical transducer or circuitry **23,** and a signal output, e.g., via leads **16** and/or a wireless transmitter.

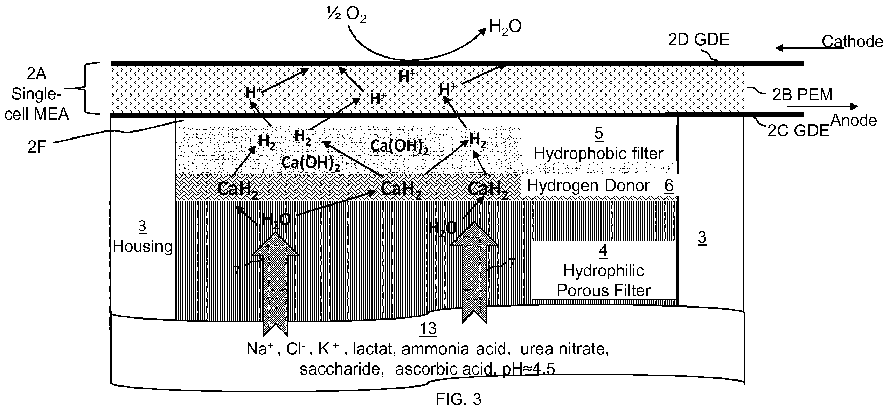

**FIG. 3** depicts an embodiment of sensor **1** applied as a sweat gauge mounted to sweating skin **13.**

Water and other sweat constituents are captured by primary hydrophilic porous filter **4,** the non-aquatic constituents filtered, and the remaining water content conveyed downstream where it contacts the hydrogen donor CaH₂ **6** disposed at the downstream edge of primary filter **4.** There the CaH₂ reacts with water to form a stoichiometric volume of hydrogen that creates a pressure gradient driving the hydrogen through secondary filter **5.** Water filter **5** filters unreacted water, CaH₂ and Ca(OH)₂ and has a low flow resistance relative to primary filter **4.** This filtering may become increasingly significant in protecting transducing element **2A** as reaction efficiency diminishes with time and the quantity of unreacted water increases. The hydrogen continues downstream and contacts transducing element **2A** implemented in this example as a single-cell membrane electrode assembly (MEA) having a proton exchange membrane PEM **2B** sandwiched between two gas diffusion electrodes GDEs **2C** and **2D,** as noted above. Each of GDEs **2C** and **2D** has an electrically conductive supporting cloth enabling gas distribution and an electrode with a catalyst where the chemical reactions occur. The catalyst coated surfaces are in contact with PEM electrolyte **2B.**

As shown, GDE **2C** hydrogen is oxidized to cations H+ and the electrons leave sensor **1** at anode **11.** The cations pass the solid-state electrolyte PEM **2B** and the oxygen is reduced and combines with cations to produce water at cathode **12,** as noted above.

The PEM **2B** is a gas selective permeable membrane resulting in a hydrogen and oxygen gradient across the membrane thickness. It acts as convey path for protons supply the GDE **2D** with protons H+, while blocking oxygen and ions thereof. The GDE **2D** in contact with the PEM promotes a high conversion rate of the protons to water.

MEA **2A** is in communication with gas distribution channel **2F** to maximize hydrogen contact with to the membrane surface. A high conversion rate is obtained by using high efficient MEAs. Non-converted excess hydrogen can leave the system after passing membrane surface **2D** in a certain embodiment.

In another embodiment, a bypass channel (not shown) directs a known, fixed fraction of hydrogen directly out of housing **3** and does not produce an electrical signal to prevent saturation of the fuel cell and facilitate miniaturization of MEA **2A.** Sensor **1** can be self-actuated and deactivated in accordance with stoichiometric limitation set by the amount of water available.

As taught, the required precision measurement is achieved though the capture of sweat in both static and flowing states and their conversion into a collective electrical signal.

Other applications include, *inter alia,* measurement of water content in concrete walls, polymeric objects, wood, and the atmosphere. Further applications are to determine the permeability of thin objects, as well as their time dependent evaporation behavior.

In yet other applications, precision measurement of water content is achievable independent of the Reynold's numbers characteristic of laminar and turbulent flow regimes.

Furthermore, in addition to its measuring capacity, the sensor also generates harnessable electricity.

Sensor **1** may be comparatively efficient and effective for static and non-static states and have a high sensitivity down to flow rates of the less than, for example, 10ul/min. Sensor **1** is also effective for liquid, gaseous, and vapor states over a temperature range like, for example, 5-40°C, has a fast response time of some seconds, has a slew rate of, e.g., 3-5 seconds to reach the nominal output power, and has a comparatively low cost. Its dynamic range spans, e.g., at least five orders of magnitude and is highly selective in that its capable of identifying water content among a variety of other substances.

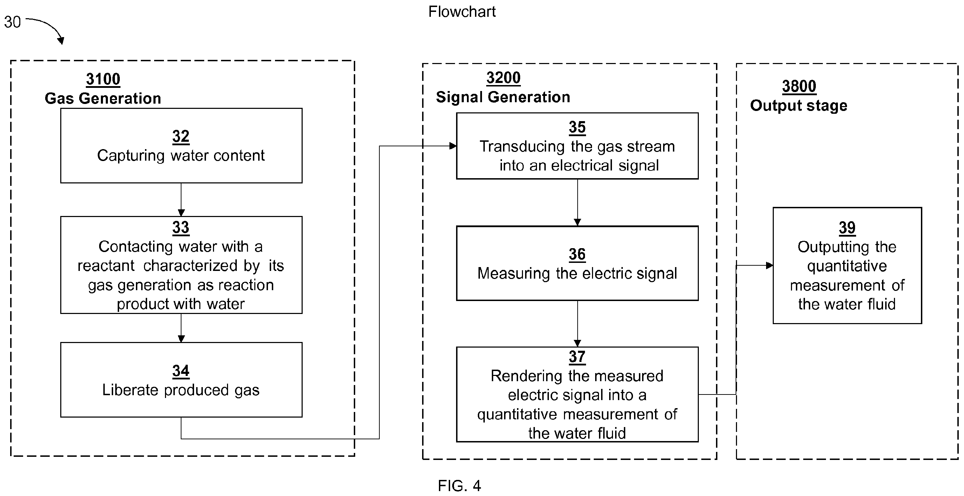

**FIG. 4** is a flowchart of the processing steps employed by the water sensor and can be divided into three stages, gas generation **3100,** signal generation **3200,** and output **3800.**

Specifically, in gas generation stage **3100,** water is captured at step **32** as a liquid, gas or vapor with a hydrophilic material, as noted above. In step **33,** the water is contacted with a reactant characterized by its gas generation properties as a reaction product. In step **34** the liberated gas is conveyed through a hydrophobic membrane **5** while filtering unreacted water and reacted CaH₂.

In stage **3200,** the liberated gas is transduced into an electrical signal at step **35** through any of the transducing element embodiments noted above. In step **36,** the signal is measured either as a current or a voltage in accordance with the type of transducing element employed. In step **37,** the measured signal is rendered into a quantitative measurement of the flow rate of water fluid in accordance with a given reaction conversion.

In the single-step output stage **3800,** the quantitative measurement is output at step **39,** for example, to a display screen.

### Additional Examples:

Examples 1 is a method for quantifying the content of water fluid, the method comprising: contacting water fluid with a reactant hydrogen donor; capturing a liberated hydrogen gas stream having a stoichiometric equivalent of the water fluid; transducing the hydrogen gas stream into an electrical signal; determining a characteristic of the water fluid in accordance with the electric signal; and providing an output descriptive of the determined water fluid characteristic.
Example 2 includes the subject matter of example 1 and, optionally, wherein the water fluid is implemented as a liquid.
Example 3 includes the subject matter of example 1 or example 2, wherein the water fluid is implemented as a gas.
Example 4 includes the subject matter of any one of the examples 1 to 3 and, optionally, wherein the water fluid is implemented as a vapor.
Example 5 includes the subject matter of any one of the examples 1 to 4 and, optionally, wherein the reactant is implemented as metallic or non-metallic hydride.
Example 6 includes the subject matter of example 5 and, optionally, wherein the metallic hydride is selected from the group consisting of MgH₂, NaAlH₄, LiAlH₄, LiH, LiBH², and LiBH₄.
Example 7 includes the subject matter of example 5 or 6 and, optionally, wherein the non-metallic hydride is implemented as CaH₂.
Example 8 includes the subject matter of any one of the examples 1 to 7 and, optionally, wherein the transducing the hydrogen stream into an electrical signal is implemented by driving a fuel cell with the hydrogen stream so as to yield a corresponding change in current flow.
Example 9 includes the subject matter of any one of the examples 1 to 8 and, optionally, wherein the transducing the hydrogen stream into an electrical signal is implemented by permeating a porous dielectric with the hydrogen stream so as to yield a corresponding change in electrical capacitance of the porous dielectric. Additionally, one can use the change of the thermal conductivity by using a low conductive porous material; (e.g. aerogel, porous silica).
Example 10 includes the subject matter of example 9 and, optionally, wherein the porous dielectric is implemented as a zeolite.
Example 11 includes an electrochemical sensor (1) for quantifying water content, the sensor (1) comprising a reactor (2) including a reactant hydrogen donor, wherein the reactor (2) is configured to liberate a hydrogen stream having a stoichiometric equivalent to water; the reactant hydrogen donor having an ability to liberate hydrogen gas upon reaction with water. The sensor (1) may further include, optionally, a transducing element configured to transduce the hydrogen stream into an electrical signal; circuitry configured to determine a quantity of the water fluid in accordance with the electrical signal; and an output device configured to output the quantity of the water fluid. Such output may comprise a visual, audible, haptic, or digital output, or a combination of them in accordance with the particular embodiment.
Example 12 includes the subject matter of example 11 and, optionally, wherein the water fluid is implemented as liquid, gas, and/or vapor.
Example 13 includes the subject matter of examples 11 or 12 and, optionally, wherein the reactant hydrogen donor is implemented as metallic or non-metallic hydride.
Example 14 includes the subject matter of any one of the examples 11 to 13 and, optionally, wherein the metallic hydride is selected from the group consisting of MgH2, NaAlH4, LiAlH4, LiH, LiBH2, and LiBH4.
Example 15 includes the subject matter of example 13 and, optionally, wherein the metallic hydride is implemented as CaH₂.
Example 16 includes the subject matter of any one of the examples 11 to 15 and, optionally, wherein the transducing element is implemented as a fuel cell driven by the hydrogen stream.
Example 17 includes the subject matter of example 16 and, optionally, wherein the fuel cell includes a proton-exchange membrane (PEM).
Example 18 includes the subject matter of any one of the examples 11 to 17 and, optionally, wherein the transducing element is implemented as porous dielectric whose capacitance changes in accordance with a degree of permeation of the hydrogen stream.
Example 19 includes the subject matter of example 18 and, optionally, wherein the porous dielectric is implemented as a zeolite.
Example 20 includes the subject matter of any one of the examples 11 to 19 and, optionally, wherein the transducing element is implemented as a pressure sensor configured to generate the electric signal responsively to a differential pressure generated by the hydrogen stream.
Example 21 includes the subject matter of any one of the examples 11 to 20, and optionally, wherein the transducing element is implemented as an anemometer configured to generate the electric signal in accordance with gas flow of the hydrogen stream.
Example 22 concerns the use of an electrochemical sensor according to any one of the examples 11 to 21.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Unless otherwise specified, the terms 'about' and/or 'close' with respect to a magnitude or a numerical value may imply to be within an inclusive range of -10% to +10% of the respective magnitude or value.

"Coupled with" means indirectly or directly "coupled with".

It should be appreciated that combination of features disclosed in different embodiments, examples, and/or the like, are also included within the scope of the present invention.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A method for quantifying a characteristic related to water, the method comprising:
contacting water fluid with a reactant gas donor;
capturing a liberated hydrogen gas stream having a stoichiometric equivalent of the water fluid;
transducing the hydrogen gas stream into an electrical signal;
determining a characteristic of the water fluid in accordance with the electric signal; and
providing an output descriptive of the determined water fluid characteristic.

2. The method of claim 1, wherein the water fluid is implemented as a liquid, vapor and/or a gas.

3. An electrochemical sensor (1) for measuring a characteristic related to water, comprising:
a reactor (2) comprising a reactant gas donor (6), wherein the reactor (2) is configured to liberate a hydrogen stream having a stoichiometric equivalent to water, the reactant gas donor (6) having an ability to liberate hydrogen gas upon reaction with water; and
a transducing element (3) configured to transduce the hydrogen stream into an electrical signal.

4. The electrochemical sensor (1) of claim 3, further comprising circuitry (20, 21, 22, 23) that is configured to determine a value related to a characteristic of water fluid in accordance with the electrical signal; and an output device configured to output the quantity of the water fluid.

5. The electrochemical sensor (1) of claim 3 or 4, wherein the water fluid is implemented as liquid, gas, and/or vapor.

6. The electrochemical sensor (1) of any one of the claims 3 to 5, wherein the reactant gas donor (6) is implemented as metallic or non-metallic hydride.

7. The electrochemical sensor (1) of claim 6, wherein the metallic hydride is selected from the group consisting of MgH2, NaAlH4, LiAlH4, LiH, LiBH2, and LiBH4.

8. The electrochemical sensor (1) of claim 7, wherein the metallic hydride is implemented as CaH2.

9. The electrochemical sensor (1) of any one of claims 3 to 8, wherein the transducing element (3) is implemented as a fuel cell (2A, 2B) driven by the hydrogen stream.

10. The electrochemical sensor (1) of claim 9, wherein the fuel cell (2A, 2B) includes a proton-exchange membrane (PEM).

11. The electrochemical sensor (1) of any one of claims 3 to 10, wherein the transducing element (3) is implemented as porous dielectric (19) whose capacitance changes in accordance with a degree of permeation of the hydrogen stream.

12. The electrochemical sensor (1) of claim 11, wherein the porous dielectric (19) is implemented as a zeolite.

13. The electrochemical sensor (1) of any one of claims 3 to 12, wherein the transducing element (3) is implemented as a pressure sensor configured to generate the electric signal responsively to a differential pressure generated by the hydrogen stream.

14. The electrochemical sensor (1) of any one of claims 3 to 13, wherein the transducing element (3) is implemented as an anemometer configured to generate the electric signal in accordance with gas flow of the hydrogen stream.

15. Use of any one of the electrochemical sensors (1) of claims 3 to 14.
